# EUROPEAN PATENT SPECIFICATION

(11) **EP 1 733 707 B1**
(45) Date of publication and mention of the grant of the patent: **03.09.2008**
(21) Application number: 05105169.6
(22) Date of filing: 13.06.2005
(51) Int. Cl.: A61F 9/007, A61B 17/32

(54) **Infusion Cannula System**
Kanüleninfusionssystem
Systeme de perfusion par cannule

(43) Date of publication of application: 20.12.2006
(73) Proprietor: Alcon, Inc., 6331 Hünenberg (CH)
(72) Inventor: Attinger, Juerg, CH-8260, Stein am Rhein (CH)
(74) Representative: Hanna, Peter William Derek

(56) References cited:
- EP-A- 0 791 336
- US-A- 4 200 106
- US-A- 4 674 502
- US-A- 5 947 990
- US-A1- 2002 038 122

## Description

### Background of the Invention

This invention relates generally to the field of microsurgery and, more particularly, to ophthalmic microsurgery.

During ophthalmic microsurgery, it is often necessary to remove, dissect, cut, delaminate, coagulate or otherwise manipulate delicate tissues within the eye. Microsurgical tools, such as vitreous cutters, infusion cannulas, micro scissors, micro forceps, illuminated laser probes and other devices are generally used. These devices generally are inserted through one or more surgical incisions in the sclera, a sclerotomy, and different tools may be used during different parts of the surgical procedure. The repeated insertion and removal of these instruments can allow vitreous humor and fluids to escape the eye out through the sclerotomy, increasing the potential for softening of the globe, bleeding, traction on the retina and/or introduction of bacteria into the eye as well as increased healing time. Accordingly, during ophthalmic surgery, infusion cannulas are often used to infuse an irrigating solution, such as a balanced salt solution, into the globe to maintain suitable pressure within the globe. Prior art infusion cannulas generally are inserted into the eye by first making an incision into the eye with a knife of lance. The infusion cannula is then inserted into the incision using a forceps because of the extremely small size of infusion cannula (on the order of 0.5 mm to 1.0 mm in diameter). The infusion supply line needs to be primed and then is attached, once again using a pair of forceps due to the small size cannula and the infusion line. Only then can the infusion line and infusion cannula be activated.

US-4,674,502 (CooperVision, Inc.), in particular Fig. 9 thereof, describes a cannula sleeve and cap arrangement for providing an axial orifice for a surgical instrument having a cutting probe together with an angled side orifice for an irrigation fluid inlet, which is secured to the distal end of a surgical handpiece, generally including the features of the precharacterizing part of claim 1 which follows.

Therefore, a need continues to exist for a simple, inexpensive combination of infusion cannula and insertion instrument.

### Brief Summary of the Invention

The present invention improves upon the prior art by providing an infusion cannula preinstalled on a surgical lance, in accordance with claims which follow. The infusion cannula includes a "Y" or "T" fitting to which an infusion line is connected. Such a combination eliminates the need for separate handling and installation of the infusion cannula and line, and provides for a simplified insertion of the infusion cannula into an eye.

Accordingly, one objective of the present invention is to provide a simple, inexpensive infusion cannula.

Another objective of the present invention is to provide a simple, inexpensive infusion cannula that is easy to install.

Another objective of the present invention is to provide a simple, inexpensive infusion cannula that is preinstalled in an installation lance.

Still another objective of the present invention is to provide a simple, inexpensive infusion cannula having a preinstalled infusion line.

These and other advantages and objectives of the present invention will become apparent from the detailed description and claims that follow.

### Brief Description of the Drawing

FIG. 1 is a perspective view of the infusion cannula system of the present invention.
FIG. 2 is a cross-sectional view of the infusion cannula system of the present invention.
FIG. 3 is an enlarged cross-sectional view of the infusion cannula of the present invention.
FIG. 4 is an enlarged cross-sectional view of the infusion cannula of the present invention taken at circle 3 in FIG. 2 illustrating the insertion of the infusion cannula into an eye.

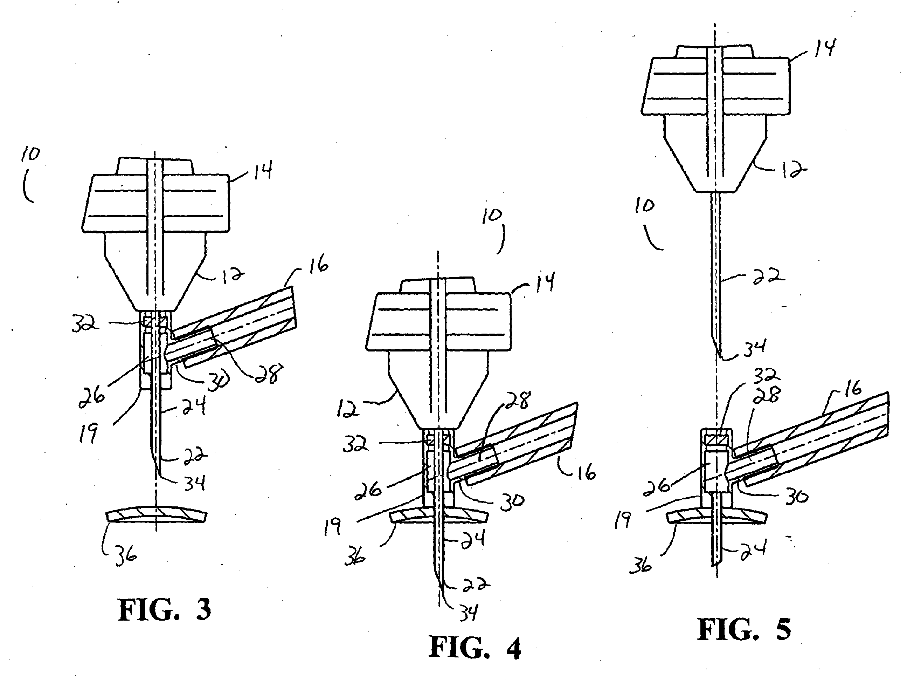

FIG. 4 is an enlarged cross-sectional view of the infusion cannula of the present invention illustrating the removal of the installation lance from the infusion cannula.

### Detailed Description of the Invention

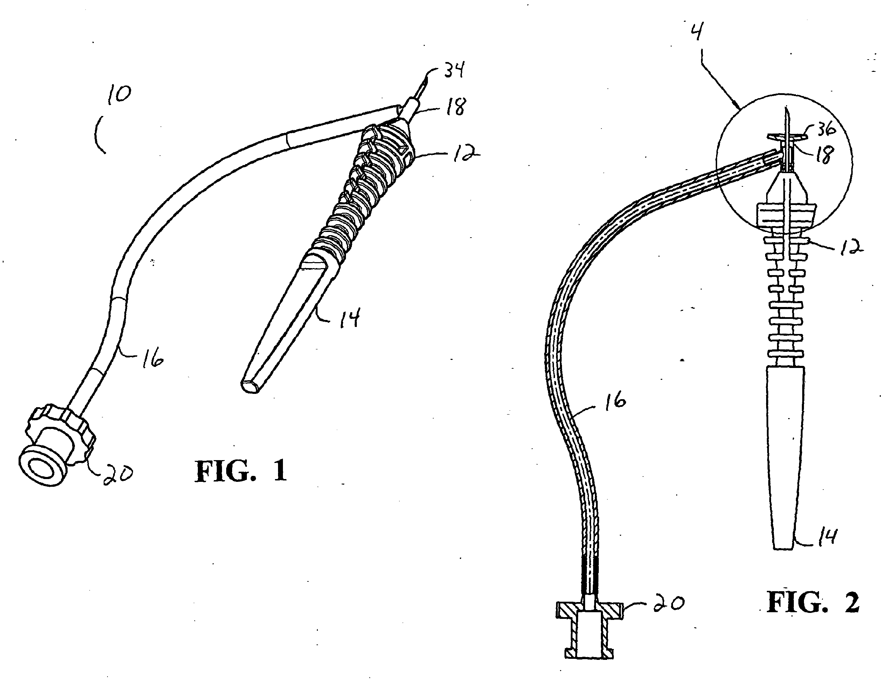

As best seen in FIGS. 1 and 2, infusion cannula system 10 of the present invention generally include lance 12 having handle 14, infusion line 16 and infusion cannula 18. Infusion line 16 is fluidly connected to infusion cannula 18 on one end, and contains a suitable connector 20, such as a luer fitting, on the opposite end for connection to a source of irrigation or infusion fluid. As best seen in FIGS 3-5, infusion cannula 18 is generally defined by hollow, tube-like body 18 defining central, longitudinal bore 26 that fluidly communicates with tube 24 of infusion cannula 18. Infusion cannula 18 further contains side bore 28, which intersects and communicates with bore 26. Side bore 28 is defined by fitting 30, which is sized and shaped to receive infusion line16. The fitting 30 may be a "Y" or "T" fitting to which an infusion line 16 is connected. Such a combination eliminates the need for separate handling and installation of the infusion cannula and line, and provides for a simplified insertion of the infusion cannula into an eye.

Lance 12 contains blade 22 sized to tightly fit within tube 24 of infusion cannula 18 so as to seal tube 24 when blade 22 is installed within infusion cannula 18 yet still be removable. Blade 22 is inserted into bore 26 and tube 24 of infusion cannula 18 through puncturable seal 32 that seals bore 26 when blade 22 is removed from infusion cannula 18. Preferably, blade 22 and tube 24 are on the order of 0.5 mm to 1.0 mm in diameter.

In use, infusion cannula assembly 10 comes preassembled with blade 22 of lance 12 inserted through bore 26 and tube 24 of infusion cannula 18 so that sharp tip 34 of blade 22 projects out of tube 24 and with infusion line 16 installed on fitting 30. Tip 34 is pressed against eye 36 so that tip 34 punctures eye 36 and blade 22 and tube 24 enters eye 36, as seen in FIG. 4. Blade 22 of lance 18 is then removed from infusion cannula 18, as shown in FIG. 5, thereby unsealing tube 24 placing bore 26 and bore 28 in fluid communication with the interior of eye 36 through tube 24. Infusion fluid from infusion line 16 can now be introduced into eye 36 with no further manipulations of infusion cannula 18 and without the need to make further connections to infusion cannula 18. Infusion fluid is prevented from exiting out of infusion cannula 18 by seal 32.

This description is given for purposes of illustration and explanation. It will be apparent to those skilled in the relevant art that changes and modifications may be made to the invention described above without departing from its scope.

## Claims

1. An infusion cannula system (10), comprising:
a) an infusion cannula (18) having a hollow body defining a central bore (26) connected on one end to a tube (24), the body further containing a fitting (30) defining a side bore (28) intersecting the central bore, the fitting in fluid communication with the tube;
b) a lance (12) having a blade (22), the blade sized to fit into the central bore (26) and the tube (24) of the infusion cannula; and
c) an infusion line (16) connectable to the fitting (30),
**characterized in that** the tube (24) is located on one side of the central bore (26) and the other side of the central bore is closed by a puncturable seal (32).

2. The infusion cannula system of claim 1, wherein the lance (12) is inserted into the tube (24) through the central bore (26) and the seal (32) so as to project out of the tube.

3. The infusion cannula system of claim 1 or claim 2, wherein the blade (22) is removably retained by the central bore (26) and the tube (24) of the infusion cannula.

4. The infusion cannula system of any one of claims 1 to 3, wherein the blade (22) is on the order of 0.5 mm to 1.0 mm in diameter.

5. The infusion cannula system of any one of claims 1 to 4, wherein the infusion line (16) is connectable at its end opposite the fitting (30) to a luer connector (20).

## Patentansprüche

1. Infusionskanülensystem (10), welches umfasst:
a) eine Infusionskanüle (18) mit einem Hohlkörper, der eine zentrale Bohrung (26) definiert, welche an einem Ende mit einem Röhrchen (24) verbunden ist, wobei der Körper weiters ein Anschlussstück (30) enthält, das eine Seitenbohrung (28) definiert, welche mit der zentralen Bohrung zusammenläuft, wobei das Anschlussstück mit dem Röhrchen in FluidVerbindung steht;
b) eine Lanze (12) mit einer Schneide (22), wobei die Schneide größenmäßig so bemessen ist, dass sie in die zentrale Bohrung (26) und in das Röhrchen (24) der Infusionskanüle passt; und
c) eine Infusionsleitung (16), die mit dem Anschlussstück (30) verbindbar ist,
**dadurch gekennzeichnet, dass** sich das Röhrchen (24) an einer Seite der zentralen Bohrung (26) befindet und die andere Seite der zentralen Bohrung durch einen durchstechbaren Verschluss (32) verschlossen ist.

2. Infusionskanülensystem nach Anspruch 1, wobei die Lanze (12) in das Röhrchen (24) durch die zentrale Bohrung (26) und den Verschluss (32) eingeführt wird, so dass sie aus dem Röhrchen herausragt.

3. Infusionskanülensystem nach Anspruch 1 oder Anspruch 2, wobei die Schneide (22) durch die zentrale Bohrung (26) und das Röhrchen (24) der Infusionskanüle entfernbar gehalten ist.

4. Infusionskanülensystem nach einem der Ansprüche 1 bis 3, wobei die Schneide (22) einen Durchmesser in der Größenordnung von 0,5 mm bis 1,0 mm hat.

5. Infusionskanülensystem nach einem der Ansprüche 1 bis 4, wobei die Infusionsleitung (16) an ihrem Ende gegenüber dem Anschlussstück (30) mit einem Luer-Verbinder (20) verbindbar ist.

## Revendications

1. Système (10) de canule de perfusion comprenant :
a) une canule (18) de perfusion ayant un corps creux définissant un alésage central (26) relié à une extrémité à un tube (24), le corps contenant en outre un raccord (30) définissant un alésage latéral (28) coupant l'alésage central, le raccord étant en communication de fluide avec le tube;
b) une lancette (12) ayant une lame (22), la lame étant d'une taille lui permettant de s'adapter à l'alésage central (26) et au tube (24) de la canule de perfusion ; et
c) une ligne (16) de perfusion pouvant être reliée au raccord (30),
**caractérisé en ce que** le tube (24) est situé sur un côté de l'alésage central (26) et l'autre côté de l'alésage central est fermé par un joint (32) pouvant être perforé.

2. Système de canule de perfusion selon la revendication 1, dans lequel la lancette (12) est insérée dans le tube (24) à travers l'alésage central (26) et le joint (32) de manière à dépasser du tube.

3. Système de canule de perfusion selon la revendication 1 ou la revendication 2, dans lequel la lame (22) est retenue de manière amovible par l'alésage central (26) et le tube (24) de la canule de perfusion.

4. Système de canule de perfusion selon l'une quelconque des revendications 1 à 3, dans lequel la lame (22) a un diamètre de l'ordre de 0,5 mm à 1,0 mm.

5. Système de canule de perfusion selon l'une quelconque des revendications 1 à 4, dans lequel la ligne de perfusion (16) peut être reliée à son extrémité opposée au raccord (30) à un connecteur luer (20).
